# EUROPEAN PATENT APPLICATION

(11) **EP 2 494 963 A1**
(43) Date of publication of application: **05.09.2012**
(21) Application number: 12157992.4
(22) Date of filing: 02.03.2012
(51) Int. Cl.: A61K 9/20, A61K 31/41, A61K 31/4422, A61K 45/06, A61K 9/14, A61P 9/12

(54) **Combinations of valsartan and amlodipine**

(30) Priority: 03.03.2011 TR 201102067
(71) Applicant: Sanovel Ilaç Sanayi Ve Ticaret Anonim Sirketi, 34460 Sariyer/Istanbul (TR)
(72) Inventor: Cifter, Ümit, 34398 Istanbul (TR); Türkyilmaz, Ali, 34398 Istanbul (TR); Saydam, Mehtap, 34398 Istanbul (TR); Tombayoglu, Vildan, 34398 Istanbul (TR)
(74) Representative: Sevinç, Erkan

(57) **Abstract**

The present invention provides a pharmaceutical tablet combination formulation, characterized by comprising valsartan or a pharmaceutically acceptable salt thereof with a mean particle size (d50) in the range of 15 to 60 µm and amlodipine or a pharmaceutically acceptable salt thereof with a mean particle size (d50) in the range of 2 to 20 µm.

## Description

### Field of Invention

The present invention relates to compositions comprising valsartan or a pharmaceutically acceptable salt thereof and amlodipine or a pharmaceutically acceptable salt thereof.

### Background of Invention

Valsartan is a specific angiotensin II receptor antagonist, which selectively and competitively blocks AT1-receptors and mediates the vasopressor and aldosterone affects of angiotensin II. Its chemical designation is N-(1-oxopentyl)-N-[[2'-(1H-tetrazol-5-yl)-[1,1'-biphenyl]-4-yl]methyl]-L-valine with the following chemical structure of Formula I.

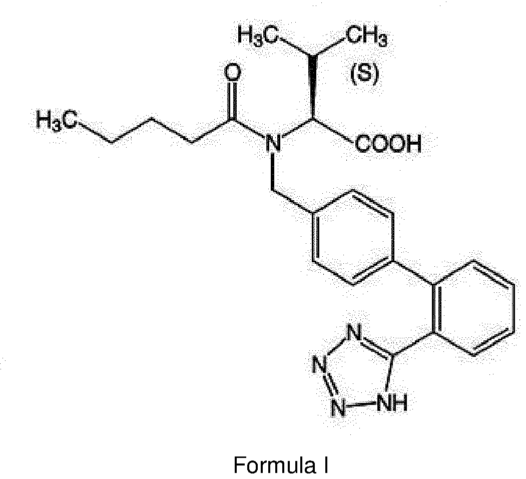

The valsartan molecule is used in treating hypertension. Valsartan is already available in 80, 160 and 320 mg tablets for oral administration.

Amlodipine is an orally-administered calcium channel blocker. Its chemical designation is (RS)-3-ethyl-5-methyl-2-(2-aminoethoxymethyl)-4-(2-chlorophenyl)-6-methyl-1,4-dihydropyridine-3,5-dicarboxylate with the following chemical structure of Formula II.

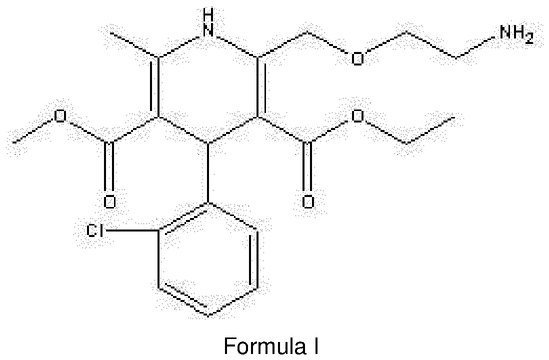

Amlodipine is presently available in the form of orally-administrable 5 to 10 mg tablets.

There have been various applications present in the patent relation for the combinations of valsartan and amlodipine.

The patent application W02007022113 discloses a solid formulation composed of valsartan, amlodipine and excipients.

The patent application W02011001440 discloses a composition composed of valsartan with mannitol and povidone. According to that application, it is also possible to add hydrochlorothiazide or amlodipine into the formulation.

The patent application US20110028456 claims a composition made of an active agent such as valsartan and/or amlodipine among the others, with a water-soluble or water-insoluble polymer.

The patent application W003097045 discloses a composition and kit of parts comprising valsartan, hydrochlorothiazide and amlodipine.

The patent application W02010081824 discloses a composition comprising a group from which enantiomerically-pure (S) and valsartan is selected.

One of the conventional tablet production methods is the direct compression method. While the production via direct compression has various difficulties, it is economic and has a high process speed. Production based on the direct compression method is affected by mixing the active agent(s) and excipient(s) used in the tablet formulation and then compressing this mixture under a properly-selected force level. The particle size of active agents and excipients is quite important in terms of the direct compression process. The causes that complicate this method are the lower amounts of active agents used, and the poor flowability and compressibility features of granules and particles.

Another important point in producing tablets is uniformly mixing the active agents that compose a formulation. It is thus ensured that any dosage form comprises the desired amount of active agent. In order to ensure a uniform mixing and to prevent any particle segregation, the difference in the particle size of the powders, pellets, and granules should not be large or should be kept within certain proportions. On the other hand, although the required particle size for direct compression does vary among the active agents and excipients, it must be kept above certain limits. If this size is not at a desired level, the direct compression process cannot be thoroughly achieved. Additionally, it should particularly be ensured that the particle size does not affect the solubility and dissolution rate parameters. Taking all these points into consideration, it may be concluded that the direct compression method for producing combinations of valsartan and amlodipine has significant difficulties. It is hereby preferred to achieve a uniform mixing and to prepare this mixture by means of an economic method such as direct compression which is advantageous in terms of the process speed.

In result, a improved and modified formulations, which can be used by hypertension patients are needed in the relevant art.

### Object of the Invention

The present invention provides an easily-administrable combination compositions of valsartan and amlodipine, eliminating all aforesaid problems and bringing additional advantages to the relevant prior art.

Accordingly, the main object of the present invention is to obtain a combinations and composition which have desired degree of uniformity in terms of its ingredients.

Another object of the present invention is to obtain an efficient and stable compositions for use in the treatment of hypertension.

A further object of the present invention is to obtain a combination of valsartan and amlodipine with proper particle sizes, which allows both to conduct a direct compression process, and to achieve a uniform mixing.

Yet a further object of the present invention is to obtain a combination of valsartan and amlodipine having desired levels of bioavailability.

Still another object of the present invention is to obtain a combination of valsartan and amlodipine having desired levels of dissolution rate and solubility.

### Detailed Description of the Invention

A novel pharmaceutical tablet composition has been developed to carry out all objects, referred to above and to emerge from the following detailed disclosure.

According to a preferred embodiment of the present invention, said novelty is realized with valsartan or a pharmaceutically acceptable salt thereof with a mean particle size (d50) within the range of 15 to 60 µm and amlodipine or a pharmaceutically acceptable salt thereof with a mean particle size (d50) within the range of 2 to 20 µm. Amlodipine, in this context, is preferably a besylate salt of amlodipine.

In a preferred embodiment according to the present invention, the mean particle size (d50) of valsartan is 20 to 50 µm and more preferably 25 to 45 µm.

In another preferred embodiment according to the present invention, the mean particle size (d50) of amlodipine is 3 to 15 µm and more preferably 5 to 10 µm.

According to the preferred embodiments the proportion of the mean particle size (d50) of valsartan to the mean particle size (d50) of amlodipine is 0.5 to 30, preferably 1 to 17, and more preferably 2 to 10.

The tablets according to the present invention, said tablet can be obtained by a direct compression method as a result of applying a force of 20 to 50 kN, preferably 25 - 45 kN, and more preferably 30 to 37 kN.

In a preferred embodiment according to the present invention, said combination further comprises at least an excipient.

In another preferred embodiment according to the present invention, said excipient comprises at least one or a mixture of diluting agents, binders, disintegrants, glidants, and lubricants.

In another preferred embodiment according to the present invention, said diluent comprises at least one or a mixture of dextrose, magnesium carbonate, magnesium oxide, sucrose, cellulose acetate, maltodextrin, lactose, microcrystalline cellulose, starch, mannitol, calcium phosphate anhydrate, calcium phosphate dihydrate, calcium phosphate trihydrate, dibasic calcium phosphate, and glucose.

In another preferred embodiment according to the present invention said binder comprises at least one or a mixture of hydroxyethyl cellulose (HEC), chitosan, carbomer, carrageenan, pectin, collagen, guar gum, gum tragacanth, shellac, carnauba wax, cetyl alcohol, stearic acid, polyamide, hydrogenated castor oil, alginic acid, beeswax, methacrylic acid/ethyl acrylate copolymer, dimethyl amino ethyl methacrylate/butyl methacrylate/metal methacrylate copolymer, hydroxypropyl methyl cellulose acetate succinate, gelatin, methacrylate derivatives, paraffin, polyvinyl acetate, cellulose acetate, cellulose phthalate, cellulose propionate, cellulose butyrate, cetostearyl alcohol, polymethacrylate, glyceryl behenate, polyvinylpyrrolidone (povidone), hydroxypropyl methyl cellulose (HPMC), hydroxypropyl cellulose (HPC), carboxymethyl cellulose (CMC), methyl cellulose (MC), ethyl cellulose and similar cellulose derivatives, polyethylene oxide, and gelatin.

In another preferred embodiment according to the present invention, said disintegrants comprises at least one or a mixture of croscarmellose sodium, sodium starch glycolate, crospovidone, starch (e.g. corn, potato), and alginic acid.

In another preferred embodiment according to the present invention, said glidant comprises at least one or a mixture of colloidal silicone dioxide, talk, aluminum silicate, and magnesium silicate.

In another preferred embodiment according to the present invention, said lubricant comprises at least one or a mixture of glyceryl behenate, glyceryl monostearate calcium stearate, magnesium stearate, mineral oil, sodium stearyl fumarate, talk, polyethylene glycol, and stearic acid.

In another preferred embodiment according to the present invention, said pharmaceutical composition consisting of
a. valsartan or a pharmaceutically acceptable salt thereof at 5 - 33% by weight,
b. amlodipine or a pharmaceutically acceptable salt thereof at 0.3-1 % by weight,
c. pregelatinized starch at 10 - 60% by weight,
d. microcrystalline cellulose at 10 - 27% by weight,
e. colloidal silicone dioxide at 0.25 - 3% by weight,
f. magnesium stearate at 0.25 - 1.5% by weight,
g. coating layer at 0.1 - 5% by weight.

Another aspect of the present invention provides a method for preparing the pharmaceutical combination according to the present invention, this method comprising the steps of
a. adding pregelatinized starch, microcrystalline cellulose, colloidal silicone dioxide and magnesium stearate into valsartan and amlodipine and mixing the resulting mixture,
b. compressing the mixture into tablets, and
c. film-coating the tablets obtained.

Another aspect of the present invention provides a method for preparing the pharmaceutical composition according to the present invention, this method comprising the steps of
a. adding 1/4 of pregelatinized starch and 1/4 of microcrystalline cellulose into amlodipine besylate, mixing the resulting mixture,
b. granulating the resulting mixture in water or mixture of water/ alcohol solution and obtaining the granules,
d. drying the granules,
e. sieving dried granules,
f. mixing the granules with valsartan, 3/4 of pregelatinized starch and 3/4 of microcrystalline cellulose
g. mixing the granules with colloidal silicon dioxide
h. mixing the granules with magnesium stearate
i. compressing the granules into tablets, and
j. film-coating the tablets.

### Example 1: Film-coated Tablet /Direct compression

| Ingredients | weight % (w/w) |
|---|---|
| core tablet | |
| valsartan | 5-33 |
| amlodipine besylate | 0.3 - 1 |
| microcrystalline cellulose PH102 | 10-27 |
| starch | 10-60 |
| colloidal silicone dioxide | 0.25 - 3 |
| magnesium stearate | 0.25 - 1.50 |
| film tablet | |
| Opadry yellow 31 F38106 | 1 - 5 |

In the tablet form prepared by direct compression according to the present invention, microcrystalline cellulose, pregelatinized starch, silicone dioxide and magnesium stearate are added into valsartan and amlodipine and tablet compression is performed accordingly. At the final step, tablets are subjected to film-coating.

### Example 2: Film-coated Tablet / Wet granulation and Direct Compression

| **Formulation** | weight % (w/w) |
|---|---|
| **Active ingredients** | |
| Amlodipine besylate | 0.3 - 1 |
| Valsartan | 5-33 |
| **Excipients** | |
| Microcrystalline cellulose | 10-27 |
| Pregelatinized starch | 10-60 |
| Colloidal silicon dioxide | 0.25 - 3 |
| Magnesium stearate | 0.25 - 1.50 |
| Opadry II Yellow (31F38122) | 1 - 5 |

In the tablet form made via wet granulation according to the present invention, amlodipine, 1/4 of pregelatinized starch and 1/4 of microcrystalline cellulose are mixed together. Then, mixture granulated in water or mixture of the water/alcohol and therefore is obtained granules. Said granules are added valsartan, 3/4 of pregelatinized starch and 3/4 of microcrystalline cellulose, the resulting mixture is mixed, colloidal silicon dioxide added and mixed, magnesium stearate is added and mixed. At the final step, the mixture is compressed into tablets and film-coated.

Valsartan is used in amounts of 80, 160 to 320 mg, and amlodipine is used in amounts of 5 to 10 mg in various combination formulations according to the present invention, which must be deemed illustrative, but not limiting.

The present invention comprises valsartan or a pharmaceutically acceptable salt of valsartan with a mean particle size (d50) within the range of 15 to 60 µm and amlodipine or a pharmaceutically acceptable salt of amlodipine with a mean particle size (d50) within the range of 2 to 20 µm. The mean particle size (d50) of valsartan is 20 - 50 µm, preferably 25 - 45 µm, whereas that of amlodipine is 3 - 15 µm and preferably 5 - 10 µm. Thanks to this, stable combination formulations of amlodipine and valsartan are obtained, which have surprisingly good bioavailability, content uniformity, and dissolution rate. Thanks to compression force and particle sizes of the active ingredients are achieved the present invention.

Amlodipine is used preferably in its besylate form in the formulation according to the present invention.

Hypertension treatment is provided with the combination formulations developed.

The protection scope of the present invention is set forth in the annexed claims and cannot be restricted to the illustrative disclosures given above, under the detailed description. Any alternative embodiments to be produced by those skilled in the art according to the basic principles, which are under the protection scope as set forth in the claims, shall be an infringement of the present invention.

## Claims

1. A pharmaceutical tablet composition, **characterized by** comprising valsartan or a pharmaceutically acceptable salt thereof with a mean particle size (d50) in the range of 15 to 60 µm and amlodipine or a pharmaceutically acceptable salt thereof with a mean particle size (d50) in the range of 2 to 20 µm.

2. The pharmaceutical tablet composition according to Claim 1, wherein the mean particle size (d50) of valsartan is 20 to 50 µm and preferably 25 to 45 µm.

3. The pharmaceutical tablet composition according to claims 1 and 2, wherein the mean particle size (d50) of amlodipine is 3 to 15 µm and preferably 5 to 10 µm.

4. The pharmaceutical composition according to any of the preceding claims, wherein amlodipine is amlodipine besylate.

5. The pharmaceutical composition according to any of the preceding claims, wherein the proportion of the mean particle size (d50) of valsartan to the mean particle size (d50) of amlodipine is 0.5 to 30, preferably 1 to 17, and more preferably 2 to 10.

6. The pharmaceutical composition according to any of the preceding claims, wherein said tablet is obtained by direct compression method as a result of applying a force of 20 to 50 kN, preferably 25 - 45 kN, and more preferably 30 to 37 kN.

7. The pharmaceutical composition according to any of the preceding claims, further comprising at least one excipient.

8. The pharmaceutical composition according to any of the preceding claims, wherein said excipient is selected from the group consisting of diluting agents, binders, disintegrants, glidants, lubricants and mixture thereof.

9. The pharmaceutical composition according to any of the preceding claims, wherein said diluent comprises at least one or a mixture of dextrose, magnesium carbonate, magnesium oxide, sucrose, cellulose acetate, maltodextrin, lactose, microcrystalline cellulose, starch, mannitol, calcium phosphate anhydrate, calcium phosphate dihydrate, calcium phosphate trihydrate, dibasic calcium phosphate, and glucose.

10. The pharmaceutical composition according to any of the preceding claims, wherein said binder comprises at least one or a mixture of hydroxyethyl cellulose (HEC), chitosan, carbomer, carrageenan, pectin, collagen, guar gum, gum tragacanth, shellac, carnauba wax, cetyl alcohol, stearic acid, polyamide, hydrogenated castor oil, alginic acid, beeswax, methacrylic acid/ethyl acrylate copolymer, dimethyl amino ethyl methacrylate/butyl methacrylate/metal methacrylate copolymer, hydroxypropyl methyl cellulose acetate succinate, gelatin, methacrylate derivatives, paraffin, polyvinyl acetate, cellulose acetate, cellulose phthalate, cellulose propionate, cellulose butyrate, cetostearyl alcohol, polymethacrylate, glyceryl behenate, polyvinylpyrrolidone (povidone), hydroxypropyl methyl cellulose (HPMC), hydroxypropyl cellulose (HPC), carboxymethyl cellulose (CMC), methyl cellulose (MC), ethyl cellulose and similar cellulose derivatives, polyethylene oxide, and gelatin.

11. The pharmaceutical composition according to any of the preceding claims, wherein said disintegrant comprises at least one or a mixture of croscarmellose sodium, sodium starch glycolate, crospovidone, starch, and alginic acid.

12. The pharmaceutical composition according to any of the preceding claims, wherein said glidant comprises at least one or a mixture of colloidal silicone dioxide, talk, aluminum silicate, and magnesium silicate.

13. The pharmaceutical composition according to any of the preceding claims, wherein said lubricant comprises at least one or a mixture of glyceryl behenate, glyceryl monostearate, calcium stearate, magnesium stearate, mineral oil, sodium stearyl fumarate, talk, polyethylene glycol, and stearic acid.

14. A pharmaceutical composition according to any of the preceding claims, consisting of,
a. valsartan or a pharmaceutically acceptable salt thereof at 5-33% by weight,
b. amlodipine or a pharmaceutically acceptable salt thereof at 0.3-1% by weight,
c. pregelatinized starch at 10 - 60% by weight,
d. microcrystalline cellulose at 10 to 27% by weight,
e. colloidal silicone dioxide at 0.25 to 3% by weight,
f. magnesium stearate at 0.25 to 1.5% by weight,
g. coating layer at 0.1 - 5% by weight.

15. A method for preparing a composition formulation according to any of the preceding claims, comprising the steps of
a. adding pregelatinized starch, microcrystalline cellulose, colloidal silicone dioxide and magnesium stearate into valsartan and amlodipine and mixing the resulting mixture,
b. compressing the mixture into tablets, and
c. film-coating the tablets obtained.

16. A method for preparing a pharmaceutical composition according to any of the preceding claims, comprising the steps of
a. adding 1/4 of pregelatinized starch and 1/4 of microcrystalline cellulose into amlodipine besylate, mixing the resulting mixture,
b. granulating the resulting mixture in water or mixture of water/ alcohol solution and obtaining the granules,
c. drying the granules,
d. sieving dried granules,
e. mixing the granules with valsartan, 3/4 of pregelatinized starch and 3/4 of microcrystalline cellulose
f. mixing the granules with colloidal silicon dioxide
g. mixing the granules with magnesium stearate
h. compressing the granules into tablets, and
i. film-coating the tablets.

17. The pharmaceutical composition according to any of the preceding claims for preventing or treating hypertension in mammalians and particularly in humans.
